# EUROPEAN PATENT SPECIFICATION

(11) **EP 2 140 902 B1**
(45) Date of publication and mention of the grant of the patent: **13.03.2013**
(21) Application number: 08305376.9
(22) Date of filing: 04.07.2008
(51) Int. Cl.: A61M 16/06

(54) **Nasal mask for treating sleep breathing disorders, such as sleep apnea**
Nasalmaske zur Behandlung von Schlafatmungsstörungen, wie z.B. Schlafapnoe
Masque nasal pour traiter les troubles de la respiration pendant le sommeil, tels que l'apnée du sommeil

(43) Date of publication of application: 06.01.2010
(73) Proprietor: Air Liquide Medical Systems S.p.A., 20148 Milano (IT)
(72) Inventor: Sandoni, Giuseppe, 25124 Brescia (BS) (IT); Rivetti, Alberto, 25038 Rovato (BS) (IT)
(74) Representative: Pittis, Olivier

(56) References cited:
- WO-A-00/78381
- WO-A-02/096342
- WO-A-2005/010608
- DE-C- 455 191
- US-A- 1 125 542

## Description

The present invention concerns a nasal mask for the administration of a gas to the nasal cavities of a user, i.e. a human being, especially for treating sleep apnea or similar sleep breathing disorders.

Continuous Positive Airway Pressure (CPAP) or Noninvasive Positive Pressure Ventilation (NPPV) can be used for treating sleep apnea or similar sleep breathing disorders.

Air or any other breathable gas is supplied by a blower or similar of an apparatus that delivers pressurized gas to a nasal mask that is arranged on the patient's face, the pressurized gas being convoyed by means of a flexible conduit linking said apparatus to the mask.

Some existing nasal masks used in such treatments of sleep breathing disorders comprise gas- delivery nozzles are designed and sized for being inserted into the nasal passages of the patient to be treated, i.e. in its nostrils.

Such nasal mask generally includes a relatively rigid body with a pair of nozzles in the form of nasal pillows, nasal prongs, cannula or nasal puffs, that are mounted on the rigid.

The nasal mask is held in place on the patient's face using a headgear assembly that can be adjusted so as to increase the comfort of the patient, in particular for taking into account the morphology of the patient's face. Examples of such nasal mask are disclosed in the following documents: WO-A-01/89381, WO-A-2004/073778, EP-A-1481702, EP-A-1317940, EP-A-1317941 and EP-A-1646910.

However, there are several problems that remain unsolved by the existing mask configurations.

A first problem is linked to the differences in terms of sizes and/or of forms of the head of several patients. Indeed, as easily understandable, a mask can perfectly match the face of a patient, i.e. leading to a sealed insertion of the nozzles in the patient's nostrils, but, in contrast, result in leaks for another patient having a different morphology.

A second problem is linked to the accumulation, in the internal body of the mask, of mucous, liquid and/or solid secretions or similar coming from the nostrils of the patient during the use of said mask. The existing masks are not easily to clean for removing these secretions and similar corporal wastes, which leads, in some cases, in a frequent need for replacement of the mask that is too dirty to be used, albeit recommended cleanings.

A third problem concerns the number and position of the gas inlets used for introduce under-pressure gas in the mask. Usually, a nasal mask comprises only one central gas inlet or two lateral gas inlets situated on each side of the mask. However, having only a central or two lateral gas inlets is not sufficient as the patient should be able to connect the tubing elements where is the most convenient or comfortable for him. In other words, it should be possible to introduce the gas, centrally or laterally, in the mask and this is not possible with the known devices.

A fourth problem is, on one hand, to avoid overpressures of gas fed into the mask and, in the other hand, to allow an efficient venting of the CO₂-containing gases expired by the patient, even if some of the venting holes are accidentally clogged, for instance, due to the position adopted by the user when sleeping.

Further, document WO-A-2005/010608 discloses a nasal mask formed by a distal element, an intermediary element with two nasal prongs to be inserted in the patient's nostrils, a sealing ring element and a proximal element. The intermediary element and the distal element define an internal volume with the sealing ring element. Connection means are provided for connecting the proximal element to the sealing ring element and the latter to the base element, and maintaining them integral during use. The sealing ring element ensures an airtight scaling between the proximal element and distal element.

In view of that, a first goal of the present invention is to provide an improved nasal mask that could be easily adapted for taking into account the morphology of the patient's.

A second goal of the invention is to provide an improved nasal mask that could be easily cleaned for removing any corporal secretions accumulated into the internal body so as to decrease the frequency of replacement of said mask, i.e. to increase its life time.

A third goal of the invention is to provide an improved nasal mask that is light to carry and easy to manufacture, especially a mask that can be made of molded parts, preferably of polymeric materials.

A fourth goal of the invention is to provide an improved nasal mask that can be used for treating various sleep breathing disorders, especially sleep apnea, and that can be connected to any existing breathing apparatus suitable for delivering a pressurized gas having a pressure and other characteristics compatible with the treatment of said sleep breathing disorders.

A fifth goal of the invention is to provide an improved nasal mask in which the gas can be introduced centrally as well as laterally.

A sixth goal of the invention is to provide an improved mask in which several venting passages are located in preferably at least two different sites, thereby providing a better controlled gas-leak and gas venting toward the exterior of the mask.

A solution according to the present invention is a nasal mask comprising a base element, a cover element comprising an opening, and an intermediary element with two nasal nozzles, said intermediary element sealingly arranged between said base element and said cover element and, with the nasal nozzles projecting outwardly through the opening, at least said intermediary element and said base element defining an internal volume for the gas, each of said nasal nozzles having an orifice in fluid communication via an internal passage with said internal volume, and connection means for connecting the cover element to the base element, and maintaining said cover element integral with said base element, characterized in that the intermediary element comprises a peripheral border as a sealing means cooperating with the base element and the cover element, said peripheral border of said intermediary element being pressed/ squeezed between the cover element and the base element..

The mask according to the present invention can further comprise one or more of the following additional features :
- the base element comprises a central opening and two lateral openings, said central opening and two lateral openings communicating with the internal volume.
- the central opening and the two lateral openings have an identical size, in particular a same diameter.
- the intermediary element comprises a peripheral border conformed like a skirt, as a sealing means cooperating with the base element and the cover element.
- the cover element comprises first connection means and the base element comprises second connection means, said first and second connection means cooperating together so as to maintain the cover element integral with at least said base element.
- a first closing element is arranged said central opening of the base element so as to prevent any gas circulation between the internal volume and the exterior of the mask via said opening.
- a second closing element is arranged in another one of said two lateral openings of the base element so as to prevent any gas circulation between the internal volume and the exterior of the mask via said opening.
- a gas-feeding element is connected to the last of said central opening and two lateral openings so as to feed gas carried by said gas-feeding element to the internal volume.
- the second closing element and/or the gas-feeding element comprise one or several venting passages for allowing a controlled gas-leak toward the exterior of the mask through said venting passages.
- the closing element and/or the gas-feeding element comprise several venting passages arranged in a circle configuration around the periphery of said elements.
- the closing element comprise several venting passages arranged in the bottom of said element.
- each of said first closing element, second closing element and gas-feeding element comprises a tubular-shaped end having an external diameter slightly less than the internal diameter of said central opening and two lateral openings of the base element so as to be able to be inserted into said openings, and comprising first fixing means cooperating with second fixing means arranged in said openings so as to maintain each said elements in position in said openings when the tubular-shaped end of said element is inserted into one of said openings.
- the first fixing means and second fixing means comprise an annular wall and an annular groove cooperating together, preferably the annular wall is arranged on the external surface of the tubular-shaped end and the annular groove is arranged in the openings.
- it further comprises a headgear assembly connected to the base element so as to maintain the mask in a desired position on a user's head when the nasal nozzles are inserted into the user's nostrils.
- the base element is rotatory connected to the headgear assembly.
- a part of the base element comprises a recess portion configured and sized for lodging at least a part of the peripheral sealing border of the intermediary element.
- the ends of said nasal nozzles carrying the orifices are conformed and sized so as to sealingly engage with the nostril conducts of a user when said nozzles are positioned in said user's nostrils, preferably the ends of said nasal nozzles have a conical or a tronconical shape.
- it is made of polymer.
- the headgear assembly comprises two elongated arms each connected, on one hand, to the base element and, on the other hand, to a headgear comprising one or several straps or similar that is positioned on the user's head.
- each of the arms of the headgear assembly comprises a flexible portion located in the vicinity of the connection site of each arm to the base element, i.e. at a distance of less than 4 cm from the connection site, preferably less than 2 cm.

A preferred embodiment of a nasal mask according to the present invention is shown on the enclosed Figures, among which :
- Figure 1 represents the nasal mask of the invention in position on the face of a patient,
- Figure 2 is an exploded view of the different parts of the mask of Figure 1,
- Figure 3 is similar to Figure 2,
- Figure 4 is an enlarged view of the mask showing the closing elements and the gas-feeding element plugged-in,
- Figures 5 and 6 are different views of the mask of Fig. 1,
- Figures 7 and 8 illustrate a particular embodiment of the gas-feeding element,
- Figure 9 is another view of the mask of Figure 4, and
- Figures 10 to 13 represent another embodiment of the headgear useable with the nasal mask of the present invention.

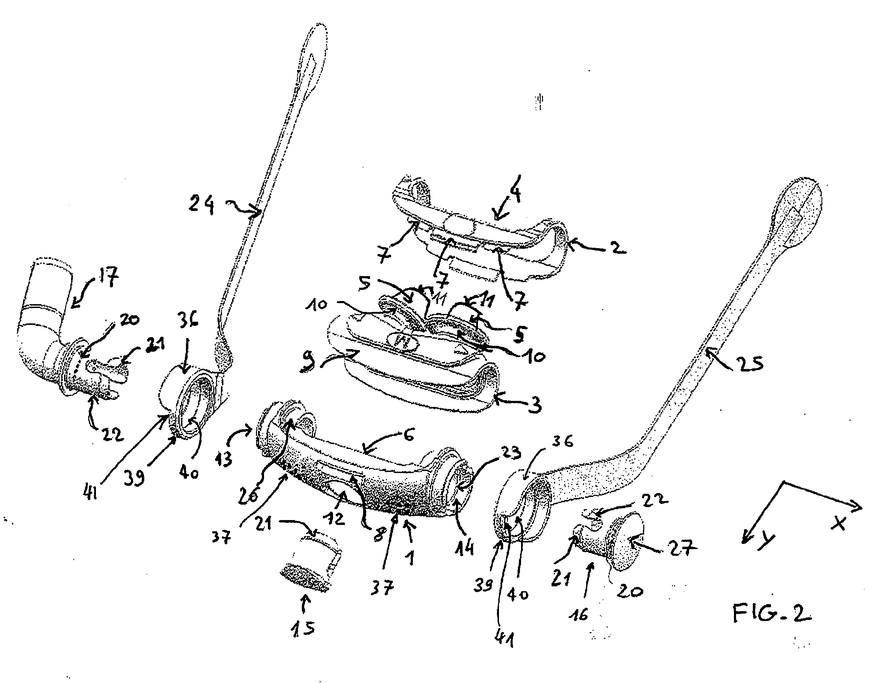

As shown on Figure 2, according to a first possible embodiment, the nasal mask of the present invention comprises a base element 1, a cover element 2 and an intermediary element 3 that is taken "in sandwich" between the base element 1 and the cover element 2.

The base element 1 forms the main body of the mask that receives the gas to be administrated to the patient's nostrils.

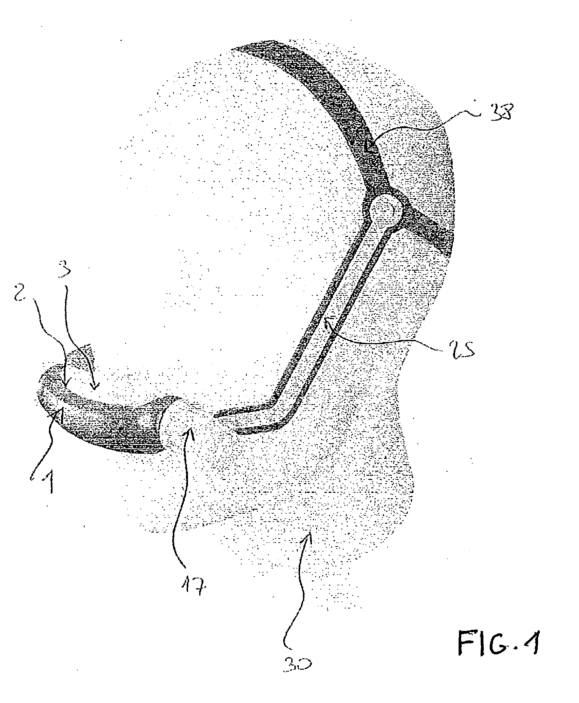

The intermediary element 3 comprises a pair of nasal nozzles 5 having each a central and internal conduct 10 for convoying the gas and an orifice 11 at their distal end, i.e. the end that has to be introduced in the patient nostrils as shown on Figure 1.

The cover element 2 comprises a central opening 4 that receives the nozzles 5 of the intermediary element 3. More precisely, the nasal nozzles 5 project outwardly through the central opening 4 of the cover element 2, when the cover element 2 is positioned and fixed on the intermediary element 3.

Further, connection means 7, 8 cooperating together are arranged on the cover element 2 and on the base element 1 for connecting the cover element 2 to the base element 1, and maintaining them integral to each other.

For instance, the cover element 2 can comprise first connection means 7, such as lips, portions of wall, recesses in wall or similar, whereas the base element 1 can comprise second connection means 8, such as lips, portions of wall, recesses in wall or similar, that cooperate together so as to maintain the cover element 2 integral with at least said base element 1, and the intermediary element 3 in a fixed position between them as explained below.

The fact that the nasal mask of the present invention comprises three elements that are detachably connected together, i.e. the base element 1, the cover element 2 and the intermediary element 3, solve several existing problems, i.e.:
- on one hand, the problems linked to the differences in terms of sizes and/or of forms of the nostrils of several patients, as the nozzles 5 are part of the intermediary element 3, said intermediary element 3 can be easily changed and replaced by another one that fits more precisely the sizes and forms of the nostrils of each patient.
- and, on the other hand, the other problems linked to the accumulation, in the internal body of the mask, of mucous, liquid and/or solid secretions or similar. Indeed, again, as the three elements 1, 2, 3 are detachably connected together, the internal parts of the mask can be easily cleaned by dissociating these three elements and cleaning them.

In order to avoid any gas leaks between these three elements 1, 2, 3 during the use of the mask, i.e. when under-pressure gas circulates in the mask body, the intermediary element 3 is sealingly arranged between the base element 1 and the cover element and 2.

The sealing function is obtained thanks to a peripheral border or skirt 9 acting as a sealing means, in particular a sealing membrane, that is arranged at the periphery of the intermediary element 3 and which cooperates with the base element 1 and the cover element and 2. More precisely, the base element 1 comprises a recess portion 26 at its periphery that is configured and sized for lodging at least a part of the peripheral border 9 of the intermediary element 3, when the intermediary element 3 is positioned on the base element 1. The cover element 2 can be positioned on the intermediary element 3 and fixed to the base element 1 by means of the connection means 7, 8, as explained above, so that the peripheral border or skirt 9 is crushed by the mechanical pressure exerted on it by the cover element 2, as the border 9 of the intermediary element 3 is taken "in sandwich" and pressed/squeezed between the cover element 2 and the recess portion 26 of the base element 1, thereby obtaining and ensuring the desired gas tightness.

As the peripheral border 9 should be crushed to get gas tightness, it is preferably formed of a soft material, such as a soft polymer or similar. The other compounds are preferably made of a rigid polymeric material in order to allow a better assembly of the different components of the mask.

Actually, the intermediary element 3 and the base element 1 are configured so as to define between them, an internal hollow volume 6 for the gas, i.e. an internal volume 6 through which the pressurized gas, such as air, introduced into the mask and inspired by the patient during the inspiration phases will circulates, as well as the CO₂-rich gas expired by the patient during the expiration phases and that should be vented to the atmosphere.

The nasal nozzles 5 of the intermediary element 3 are in fluid communication via their internal passage 10 with said internal volume 6 so to allow a circulation of gas between the internal volume 6 of the mask and the patient's nostrils, and vice versa.

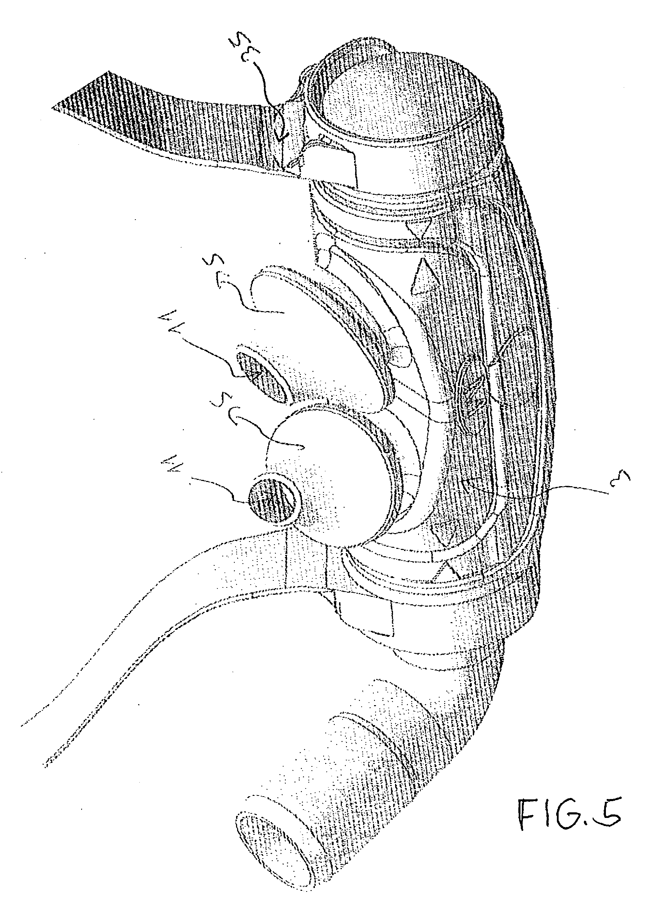

In order to ensure efficient gas exchanges, the distal ends of the nasal nozzles 5 carrying the orifices 11 are conformed and sized so as to sealingly engage with the nostril conducts of the user when said nozzles 5 are positioned in said user's nostrils, preferably the ends of said nasal nozzles 5 have a conical or a tronconical shape as represented on Figure 2 and Figure 5.

In order to address the problem linked to the number and position of gas inlets used for introducing under-pressure gas in the mask, the base element 1 comprises a central opening 12 and two lateral openings 13, 14, each communicating with the internal volume 6 so as to be able to introduce gas into the mask through any one of these openings. In other words, arranging three openings 12, 13, 14 in the base element 1 allows introducing the gas either centrally through the central opening 12, or laterally, i.e. from right or left, laterally through the lateral opening 13, 14.

Advantageously, the central opening 12 and the two lateral openings 13, 14 have an identical size, in particular a same diameter, for instance a diameter from about between 5 mm and 25 mm.

In the embodiment shown on Figure 2, a first closing element 15 is arranged in the central opening 12, whereas a second closing element 16 is arranged in one 14 of the lateral openings 13, 14 of the base element 1 so as to prevent any gas circulation between the internal volume 6 and the exterior of the mask through said central opening 12 and lateral openings 13. Further, a gas-feeding element 17, connected to a gas conduct 30 as shown on Figure 1, is connected to the remaining lateral openings 13 so as to feed gas under pressure, such as pressurized air, to the internal volume 6 of the mask body.

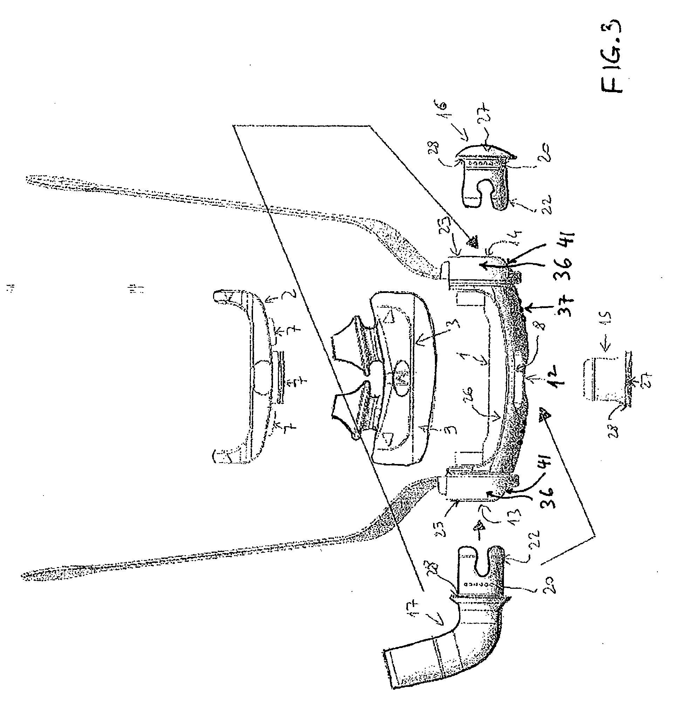

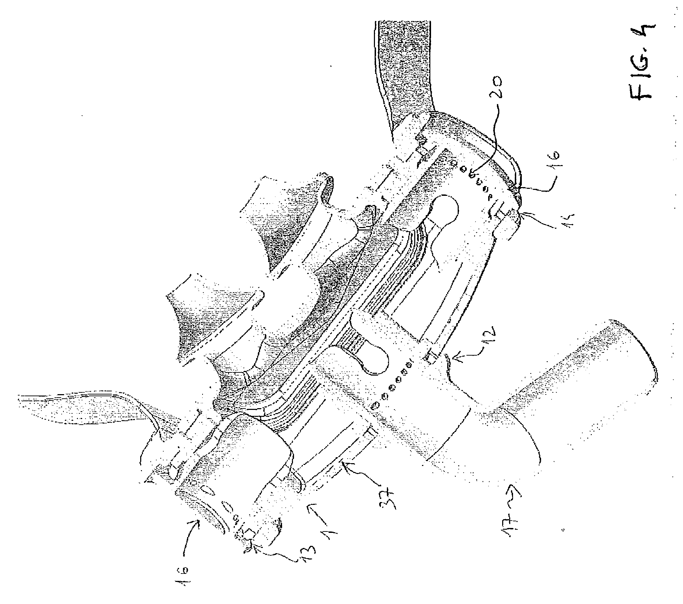

Figure 4 is similar to Figure 2 and 3, expect that, in this case, the gas-feeding element 17 has been plugged-in the central opening 12, whereas the first closing element 15 is plugged in on of the lateral openings 13, 14 of the base element 1

The closing element 15, 16 can have a shape and configuration as represented on Figure 3. In other words, they can be made of a cylindrical hollow body having a length from between 5 to 30 mm, and a diameter from about between 5 and 25 mm, which is closed at one of its ends by a bottom 27. Said bottom 27 comprises an external annular surface 28 forming for instance a kind of ring around the cylindrical hollow body that comes in abutment against the end periphery 29 of the openings 12, 13, 14 in which it is plugged in so as to limit the depth of penetration into the openings 12, 13, 14 of the cylindrical hollow body constituting the closing element 15, 16. However, the first closing element 15 and the second closing element 16 do not necessarily have exactly the same shape.

Preferably, the first closing element 15, the second closing element 16 and the gas-feeding element 17 have a similar tubular-shaped end 21 as shown on Figures 2, in order to be plugged into any one of the openings 12, 13, 14, as represented by the arrows of Figure 3.

Thus, the tubular-shaped end 21 of these plug-in components has an external diameter slightly less than the internal diameter of said central opening 12 and of the two lateral openings 13, 14 of the base element.

As shown of Figure 3, a similar external annular surface 28 is preferably arranged on the peripheral surface of the gas-feeding element 17, which has also a generally cylindrical hollow body, so that this external annular surface 28 comes in abutment against the end periphery 29 of the openings 12, 13, 14 in which the gas-feeding element 17 is plugged in and thus limit the course, i.e. the penetration length of said gas-feeding element 17 into the openings.

Of course, even if external annular surface 28 forms preferably a ring around the periphery of the closing element 15, 16 and of the gas-feeding element 17, it can have other shapes, for example formed of a discontinued ring or one or several nipples or abutments.

Anyway, to avoid over-pressures due to a too-elevated pressure level of the gas fed in the internal volume by the gas-feeding element 17 and, during expiration, to obtain an efficient venting of the expired gas at least some, but preferably all of the first closing element 15, second closing element 16 and gas-feeding element 17 comprise several venting passages or ports 37, preferably having calibrated diameter(s), that are communicating with the ambient atmosphere thus allowing a controlled gas-leak and gas venting toward the exterior of the mask through said venting passages or ports 37.

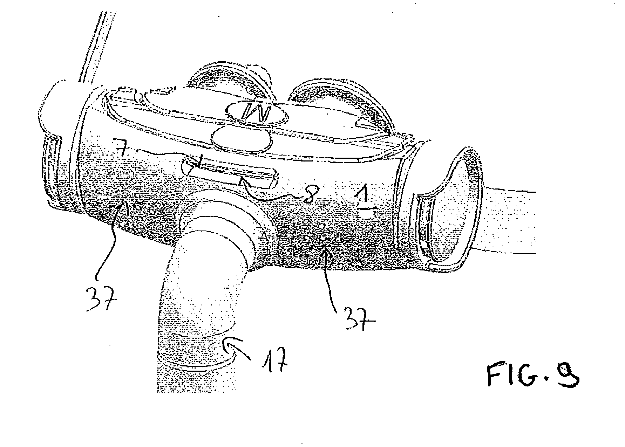

In a first embodiment, venting passages 37, preferably having a calibrated diameter, are arranged in the mask body, such as in the wall of the base element 1 as shown, for instance, on Figures 2, 4 and 9, so that the internal volume 6 can be in fluid communication with the ambient atmosphere through this/these venting passages 37.

Additional venting passages or ports 20, preferably having also a calibrated diameter, are arranged in circle(s) through the wall and around the periphery of the cylindrical hollow body of said elements 16, 17 as visible on Figure 2 and 3. Said venting passages 20 can provide an air flow, in particular when venting passages 37 are accidentally clogged, thanks to the shape of defectors 36. For instance, such venting passages 37 and 20 can have diameter from between 0.2 and 5 mm.

The additional venting passages 20 and the venting passages 37 provide a controlled gas-leak and gas venting toward the exterior of the mask.

Further, in the goal of maintaining each said components 15, 16, 17 in position in the openings 12, 13, 14, once they have been plugged-in, i.e. when the tubular-shaped end 21 of said element 15, 16, 17 is inserted into one of said openings 12, 13, 14, first fixing means 22 are on the tubular-shaped ends 21 of the plug-in components 15, 16, 17 cooperates with second fixing means 23 arranged in the openings 12, 13, 14.

Preferably, the first and second fixing means 22, 23 comprise an annular wall and an annular groove cooperating together, preferably an annular wall 22 is arranged on the periphery of the external surface of the tubular-shaped end 21 and an annular groove 23 is arranged in the wall of the openings 12, 13, 14 in such a way that each annular wall 22 enters into a corresponding annular groove 23, so as to ensure tight fastening of the plug-in components 15, 16, 17 into the openings 12, 13, 14.

For efficiently positioning and maintaining the mask in a desired position on a user's head, when the nasal nozzles 5 are inserted into the user's nostrils, the mask is equipped with a headgear assembly 24, 25, 38 connected to the base element 1 as shown on Figure 1. Preferably, the headgear assembly 24, 25, 38 is rotatory connected to the base element 1 so as to ensure a better positioning of the nozzles 5 in the nostrils of the user.

Said headgear assembly 24, 25, 38 comprises two elongated arms 24, 25 each connected, on one hand, to the base element 1 of the mask body and, on the other hand, to a headgear 38 comprising one or several straps or similar that is positioned on the user's head.

In order to increase the flexibility and the elasticity of the headgear assembly 24, 25, 38 and thus to allow a better positioning of said headgear assembly 24, 25, 38 on the user's head, the arms 24, 25 each comprise a flexible portion 35, as shown on Figure 5, located close to the connection site of each arm 24, 25 to the base element 1 of the mask body. The flexible portion 35 can be obtained in locally decreasing the thickness of the arm 24, 25, i.e. in shaping one or several grooves in these arms 24, 25. In other words, the arms 24, 25 are more rigid than the flexible portion 35 of each arm. The flexible portion 35 allows to, at least partially, solve the problem of patients having different head morphologies.

Further, the end 39 of each arm 24, 25 of the headgear assembly 24, 25, 38 are conformed to be easily coupled to the base element 1. For instance, they can have hollow circular 40 ends 39 as show on Figure 2, having a size and a shape that match the size and the shape of the extremities of the base element 1 where they are linked/connected. Such a circular or semi-circular configuration allows obtaining a rotatable connection of the headgear assembly on the base element 1.

In order to deflect the air flows vented by the additional venting passages 20 arranged around the periphery of the cylindrical hollow body of elements 15, 16, 17, as shown on Figure 3, the end 39 of each arm 24, 25 of the headgear further comprises a gas deflector 36.

The deflectors 36 are made for instance in the wall of end 39 of each arm 24, 25 as shown on Figure 2 and configured so as deflect the gas flow vented by the additional venting passages away from the user.

Preferably, the deflectors 36 are arranged such as to divert the gas flow vented through additional the venting passages 20 through the opening 41 especially when the venting passages 37 are accidentally clogged (see Fig. 2).

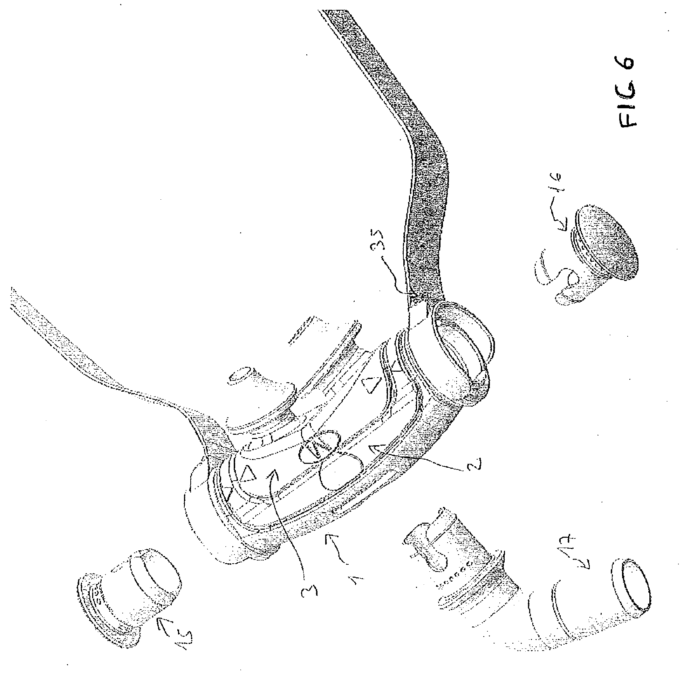

Figures 5 and 6 are different views of the mask of Fig. 1. As one can see on Figure 5, the orifices 11 of the nasal nozzles 5 have preferably an oval section, i.e. a non circular section, so as to better fit with the internal conducts of the nostrils.

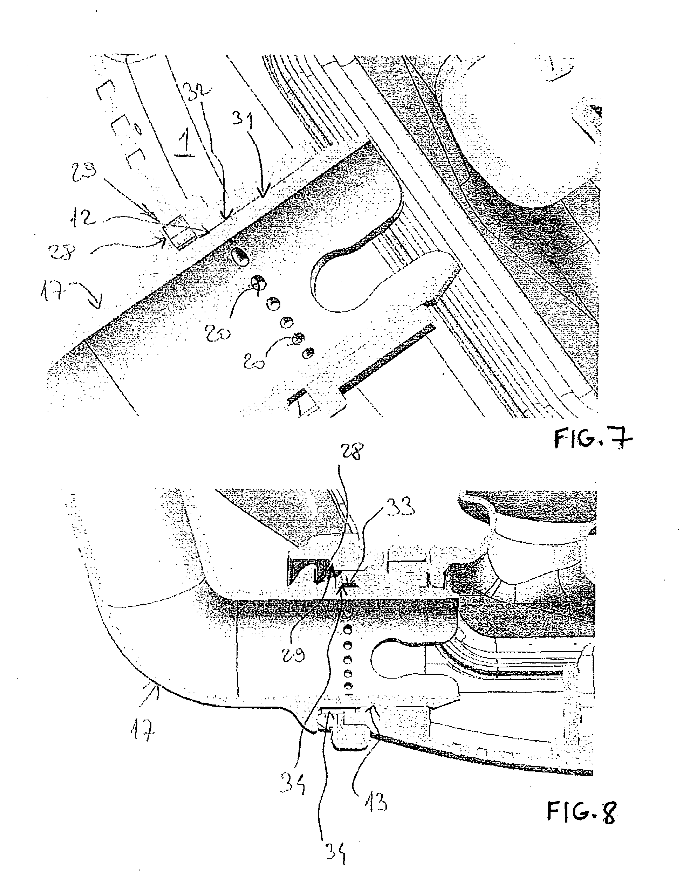

Figures 7 and 8 illustrate a particular embodiment of the gas-feeding element 17, wherein no gas is vented through the venting holes 20 of said gas-feeding element 17 when it is plugged in the central opening 12 of the base element 1 (Fig. 7), but a gas venting takes place when it is plugged in the lateral openings 13, 14 (Fig. 8).

Indeed, it could be desired, in certain cases, to prevent any gas venting via the venting holes 20 of the gas-feeding element 17 when it is plugged in the central opening 12, but not when the same element 17 is plugged in the lateral openings 13, 14.

To solve this problem, the positioning of the venting holes 20 arranged in the gas-feeding element 17, the positioning of the annular ring 28 on the external periphery of the gas-feeding element 17, and the shape of the internal wall 31 of the central opening 12 are chosen such that the venting holes 20 are closed by a portion 32 of the internal wall 31 of the central opening 12 when the gas-feeding element 17 is inserted into said central opening 12 of the base element 1 in such a way that the external annular surface 28 of the gas-feeding element 17 comes in abutment against, i.e. in contact with, the end periphery 29 of the central opening 12, thereby preventing any gas venting or leaks through the venting holes 20, i.e. from inside the gas-feeding element 17 toward the atmosphere.

In contrast, as represented on Figure 8, when the gas-feeding element 17 is plugged in either of the lateral openings 13, 14, the internal wall 33 of said lateral openings 13, 14 is configured so as to have a hollow space 34 facing the venting holes, i.e. between a portion of the internal wall 33 and the area of the periphery of the gas-feeding element 17 carrying the venting holes 20, said hollow space 34 communicating with the atmosphere through the opening 41 so that some gas can be vented to the atmosphere (see Arrow on Fig. 8).

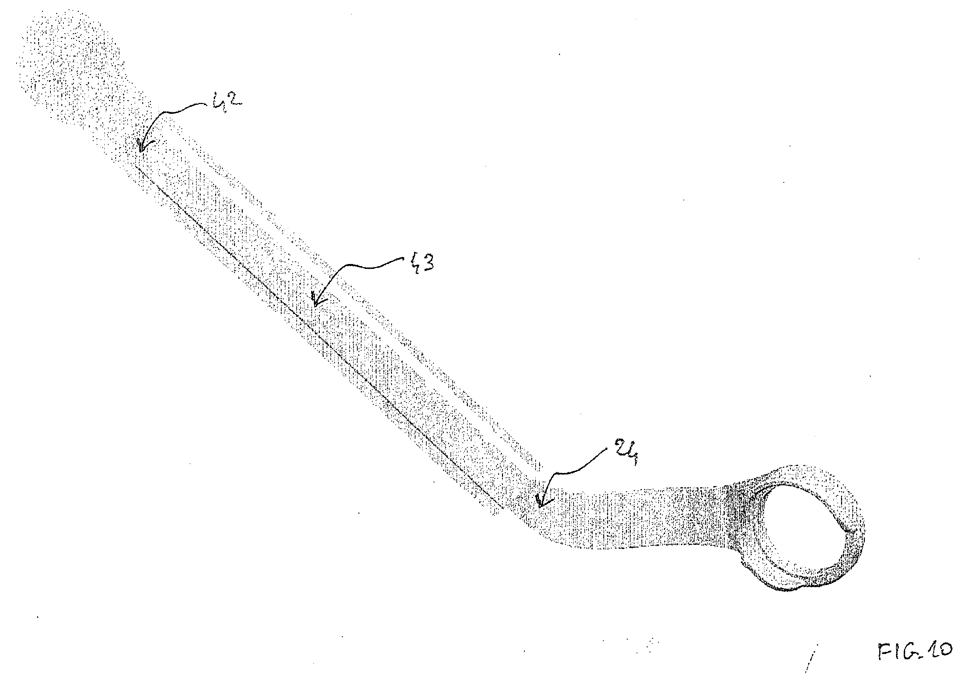

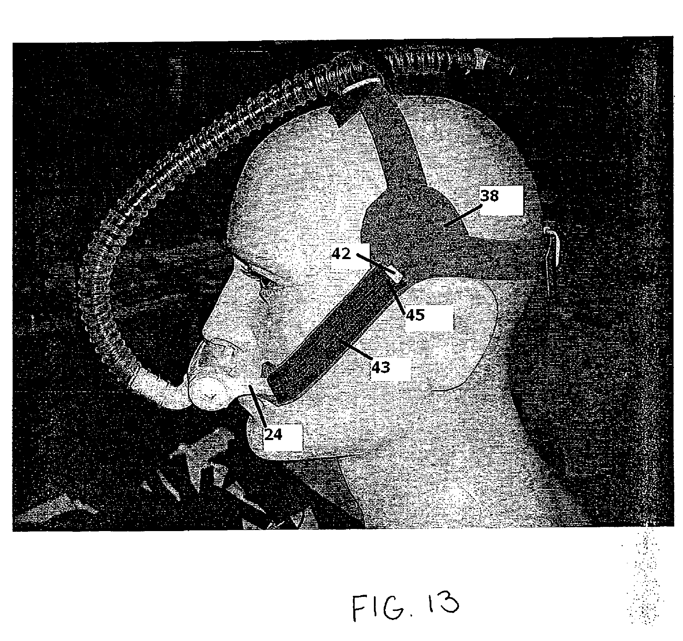

Furtermore, Figures 10 to 13 represent another embodiment of the headgear assembly 24, 25, 38 useable with the nasal mask of the present invention, wherein each arm 24, 25 is connected or linked, on one hand, to an elastic element 43 that is itself connected to the headgear 38 (see Figure 13) and, on the other hand, to the base element 1 of the mask body as in the previous embodiment of Figure 5, for instance.

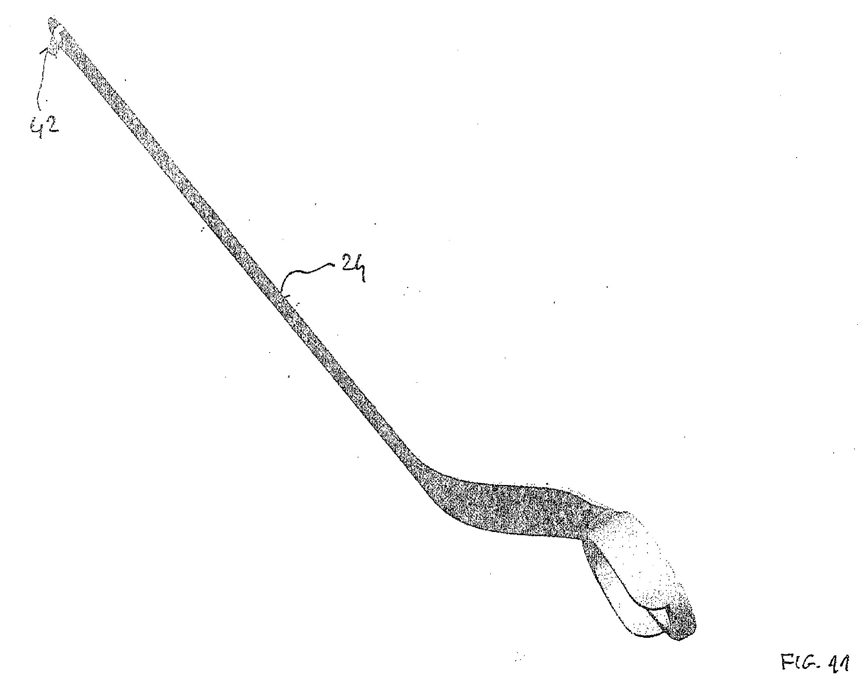

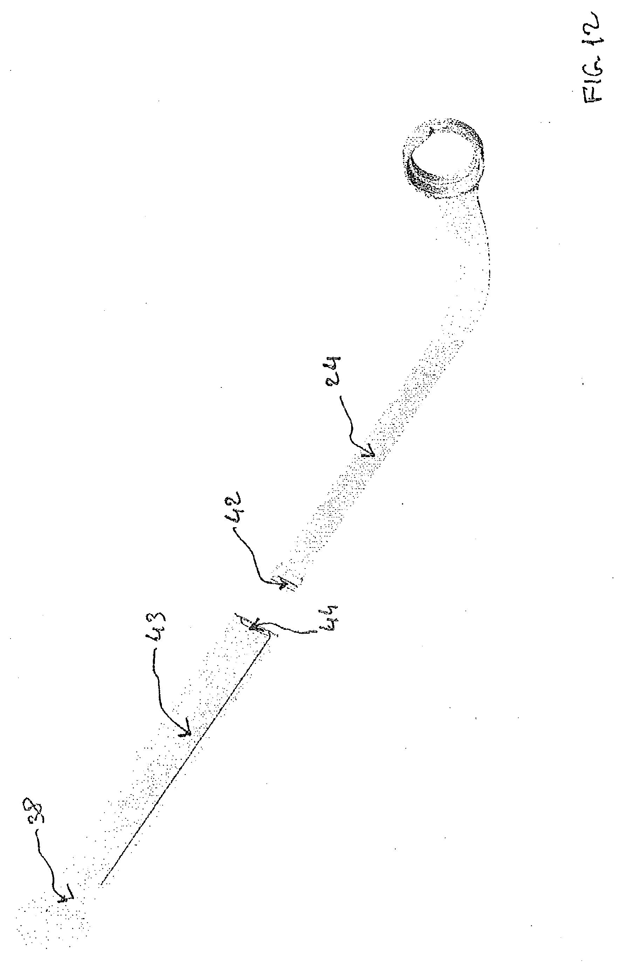

Actually, as visible on Figure 12, each elastic element 43 is elongated and hollow, and each arm 24, 25 is inserted into said hollow elastic element 43 and maintained therein by fixing means, such as a hook 42 arranged at the end 45 of each arm 24, 25, as illustrated on Figures 11 and 13.

In other words, each elastic element 43 forms a sleeve 44 that is sized to receive an arm 24, 25. The elastic element 43 can be made of an elastic or extensible fabric, band or similar, for instance a fabric comprising rubber threads.

The nasal mask of the invention can be fed with pressurized gas, such as pressurized air, delivered by a medical ventilator or similar that is adapted for the treatment of sleep apnea or similar sleep breathing disorders.

## Claims

1. Nasal mask comprising :
- a base element (1), a cover element (2) comprising an opening (4), and an intermediary element (3) with two nasal nozzles (5),
- said intermediary element (3) sealingly arranged between said base element (1) and said cover element and (2), with the nasal nozzles (5) projecting outwardly through the opening (4),
- at least said intermediary element (3) and said base clement (1) defining an internal volume (6) for the gas, each of said nasal nozzles (5) having an orifice (11) in fluid communication via an internal passage (10) with said internal volume (6), and
- connection means (7, 8) for connecting the cover element (2) to the base element (1), and maintaining said cover clement (2) integral with said base element (1),
**characterized in that** the intermediary element (3) comprises a peripheral border (9) as a sealing means cooperating with the base element (1) and the cover element (2), said peripheral border (9) of said intermediary element (3) being pressed/squeezed between the cover element (2) and the base element (1).

2. Mask according to claim 1, **characterized in that** the base element (1) comprises a central opening (12) and two lateral openings (13, 14), said central opening (12) and two lateral openings (13, 14) communicating with the internal volume (6).

3. Mask according to claim 1 or 2, **characterized in that** the central opening (12) and the two lateral openings (13, 14) have an identical size.

4. Mask according to any of the preceding claims, **characterized in that** a part (26) of the base element (1) comprises a recess portion (26) configured and sized for lodging at least a part of the peripheral sealing border (9) of the intermediary element (3).

5. Mask according to any of the preceding claims, **characterized in that** the peripheral border (9) of the intermediary element (3) is taken in sandwich and pressed/squeezed between the cover element (2) and the recess portion (26) of the base element (1).

6. Mask according to any of the preceding claims, **characterized in that** the cover element (2) comprises first connection means (7) and the base element (1) comprises second connection means (8), said first and second connection means (7, 8) cooperating together so as to maintain the cover clement (2) integral with at least said base element (1).

7. Mask according to any of the preceding claims, **characterized in that** :
- a first closing element (15) is arranged in one of said central opening (12) and two lateral openings (13, 14) of the base element (1) so as to prevent any gas circulation between the internal volume (6) and the exterior of the mask via said opening,
- a second closing element (16) is arranged in another one of said two lateral openings (13, 14) of the base element (1) so as to prevent any gas circulation between the internal volume (6) and the exterior of the mask via said opening,
- a gas-feeding element (17) is connected to the last of said central opening (12) and two lateral openings (13, 14) so as to feed gas carried by said gas-feeding element (17) to the internal volume (6).

8. Mask according to any of the preceding claims, **characterized in that** said closing element (16) and/or said gas-feeding element (17) comprise several venting passages (20) arranged in a circle configuration around the periphery of said elements (16, 17).

9. Mask according to any of the preceding claims, **characterized in that** each of said first closing element (15), second closing element (16) and gas-feeding element (17) comprises a tubular-shaped end (21) :
- having an external diameter slightly less than the internal diameter of said central opening (12) and two lateral openings (13, 14) of the base element (I) so as to be able to be inserted into said openings (12, 13, 14), and
- comprising first fixing means (22) cooperating with second fixing means (23) arranged in said openings (12, 13, 14) so as to maintain each said elements (15, 16, 17) in position in said openings (12, 13, 14) when the tubular-shaped end (21) of said element (15, 16, 17) is inserted into one of said openings (12, 13, 14).

10. Mask according to claim 9, **characterized in that** the first fixing means (22) and second fixing means (23) comprise an annular wall and an annular groove cooperating together, preferably the annular wall (22) is arranged on the external surface of the tubular-shaped end (21) and the annular groove (23) is arranged in the openings (12, 13, 14).

11. Mask according to any of the preceding claims, **characterized in that** it further comprises a headgear assembly (24, 25, 38) connected to the base element (1) so as to maintain the mask in a desired position on a user's head when the nasal nozzles (5) are inserted into the user's nostrils.

12. Mask according to any of the preceding claims, **characterized in that** the base element (1) is rotatory connected to the headgear assembly (24, 25, 38).

13. Mask according to any of the preceding claims, **characterized in that** the headgear assembly (24, 25, 38) comprises two elongated arms (24, 25) each connected, on one hand, to the base element (1) and, on the other hand, to a headgear (38) comprising one or several straps or similar that is positioned on the user's head.

14. Mask according to any of the preceding claims, **characterized in** the base element (1), the cover element and (2) and the intermediary element (3) arc detachably connected together.

15. Mask according to any of the preceding claims, **characterized in that** each of the arms (24, 25) of the headgear assembly (24, 25, 38) comprises a flexible portion (35) located in the vicinity of ends (39) of the arms (24,25).

16. Mask according to any of the preceding claims, **characterized in that** at least one gas deflector (36) is arranged at the end (39) of at least of the arms (24, 25) to deflect the gas flow vented through the additional venting passages (20), preferably the deflectors (36) are arranged such as to divert the gas flow vented through said additional venting passages (20), especially when venting passages (37) are clogged.

## Patentansprüche

1. Nasalmaske, Folgendes umfassend:
- ein Basiselement (1), ein Abdeckelement (2), das eine Öffnung (4) umfasst, und ein Zwischenelement (3) mit zwei Nasenauslässen (5),
wobei das Zwischenelement (3) zwischen dem Basiselement (1) und dem Abdeckelement (2) abdichtend angeordnet ist, und wobei die Nasenauslässe (5) durch die Öffnung (4) nach außen ragen,
wobei mindestens das Zwischenelement (3) und das Basiselement (1) ein Innenvolumen (6) für das Gas definieren und jeder der Nasenauslässe (5) eine Mündung (11) aufweist, die über einen inneren Durchlass (10) mit dem Innenvolumen (6) in Fluidverbindung steht, und
- Verbindungsmittel (7, 8), um das Abdeckelement (2) mit dem Basiselement (1) zu verbinden und das Abdeckelement (2) mit dem Basiselement (1) als Ganzes zusammenzuhalten,
**dadurch gekennzeichnet, dass** das Zwischenelement (3) eine Außenbegrenzung (9) als Dichtungsmittel umfasst, die mit dem Basiselement (1) und dem Abdeckelement (2) zusammenwirkt, wobei die Außenbegrenzung (9) des s Z wischenelements (3) zwischen dem Abdeckelement (2) und dem Basiselement (1) eingepresst ist.

2. Maske nach Anspruch 1, **dadurch gekennzeichnet, dass** das Basiselement (1) eine Mittelöffnung (12) und zwei Seitenöffnungen (13, 14) umfasst, wobei die Mittelöffnung (12) und die zwei Seitenöffnungen (13, 14) mit dem Innenvolumen (6) in Verbindung stehen.

3. Maske nach Anspruch 1 oder 2, **dadurch gekennzeichnet, dass** die Mittelöffnung (12) und die zwei Seitenöffnungen (13, 14) eine identische Größe aufweisen.

4. Maske nach einem der vorhergehenden Ansprüche, **dadurch gekennzeichnet, dass** ein Teil (26) des Basiselements (1) einen vertieften Abschnitt (26) umfasst, der dafür gestaltet und bemessen ist, mindestens einen Teil der abdichtenden Außenbegrenzung (9) des Zwischenelements (3) unterzubringen.

5. Maske nach einem der vorhergehenden Ansprüche, **dadurch gekennzeichnet, dass** die Außenbegrenzung (9) des Zwischenelements (3) zwischen dem Abdeckelement (2) und dem vertieften Abschnitt (26) des Basiselements (1) aufgenommen und eingepresst ist.

6. Maske nach einem der vorhergehenden Ansprüche, **dadurch gekennzeichnet, dass** das Abdeckelement (2) erste Verbindungsmittel (7) umfasst und das Basiselement (1) zweite Verbindungsmittel (8) umfasst, wobei die ersten und zweiten Verbindungsmittel (7, 8) zusammenwirken, um das Abdeckelement (2) und das Basiselement (1) als Ganzes zusammenzuhalten.

7. Maske nach einem der vorhergehenden Ansprüche, **dadurch gekennzeichnet, dass**:
- ein erstes Verschlusselement (15) derart in einer der Öffnungen, der Mittelöffnung (12) oder den zwei Seitenöffnungen (13, 14), des Basiselements (1) angeordnet ist, dass jeglicher Gasaustausch zwischen dem Innenvolumen (6) und dem Außenumfeld der Maske über die Öffnung unterbunden wird,
- ein zweites Verschlusselement (16) derart in einer anderen der zwei Seitenöffnungen (13, 14), des Basiselements (1) angeordnet ist, dass jeglicher Gasaustausch zwischen dem Innenvolumen (6) und dem Außenumfeld der Maske über die Öffnung unterbunden wird,
- ein Gaszufuhrelement (17) derart mit der letzten der Öffnungen, der Mittelöffnung (12) und den zwei Seitenöffnungen (13, 14), verbunden ist, dass dem Innenvolumen (6) vom Gaszufuhrelement (17) geleitetes Gas zugeführt wird.

8. Maske nach einem der vorhergehenden Ansprüche, **dadurch gekennzeichnet, dass** das Verschlusselement (16) und/oder das Gaszufuhrelement (17) mehrere Lüftungsdurchlässe (20) umfasst, die in kreisförmiger Anordnung um den Außenumfang der Elemente (16, 17) angeordnet sind.

9. Maske nach einem der vorhergehenden Ansprüche, **dadurch gekennzeichnet, dass** das erste Verschlusselement (15), das zweite Verschlusselement (16) und das Gaszufuhrelement (17) jeweils ein röhrenförmiges Ende (21) umfassen, das:
- einen Außendurchmesser aufweist, der etwas geringer ist als der Innendurchmesser der Mittelöffnung (12) und der zwei Seitenöffnungen (13, 14) des Basiselements (1), so dass es in die Öffnungen (12, 13, 14) eingeführt werden kann, und
- erste Befestigungsmittel (22) umfasst, die mit zweiten Befestigungsmitteln (23) zusammenwirken, die derart in den Öffnungen (12, 13, 14) angeordnet sind, dass jedes der Elemente (15, 16, 17) in den Öffnungen (12, 13, 14) in Position gehalten wird, wenn das röhrenförmige Ende (21) des Elements (15, 16, 17) in eine der Öffnungen (12, 13, 14) eingeführt wird.

10. Maske nach Anspruch 9, **dadurch gekennzeichnet, dass** die ersten Befestigungsmittel (22) und die zweiten Befestigungsmittel (23) eine ringförmige Wandung und eine ringförmige Rille umfassen, die zusammenwirken, wobei vorzugsweise die ringförmige Wandung (22) auf der Außenfläche des röhrenförmigen Endes (21) angeordnet ist und die ringförmige Rille (23) in den Öffnungen (12, 13, 14) angeordnet ist.

11. Maske nach einem der vorhergehenden Ansprüche, **dadurch gekennzeichnet, dass** sie ferner eine Kopfgeschirranordnung (24, 25, 38) umfasst, die derart mit dem Basiselement (1) verbunden ist, dass die Maske in einer gewünschten Position am Kopf des Benutzers gehalten wird, wenn die Nasenauslässe (5) in die Nasenlöcher des Benutzers eingeführt sind.

12. Maske nach einem der vorhergehenden Ansprüche, **dadurch gekennzeichnet, dass** das Basiselement (1) drehbar mit der Kopfgeschirranordnung (24, 25, 38) verbunden ist.

13. Maske nach einem der vorhergehenden Ansprüche, **dadurch gekennzeichnet, dass** die Kopfgeschirranordnung (24, 25, 38) zwei verlängerte Arme (24, 25) umfasst, die jeweils einerseits mit dem Basiselement (1) und andererseits mit einem Kopfgeschirr (38) verbunden sind, das einen oder mehrere Gurte oder ähnliches umfasst, das am Kopf des Benutzers angeordnet ist.

14. Maske nach einem der vorhergehenden Ansprüche, **dadurch gekennzeichnet, dass** das Basiselement (1), das Abdeckelement (2) und das Zwischenelement (3) lösbar miteinander verbunden sind.

15. Maske nach einem der vorhergehenden Ansprüche, **dadurch gekennzeichnet, dass** jeder der Arme (24, 25) der Kopfgeschirranordnung (24, 25, 38) einen flexiblen Abschnitt (35) umfasst, der in der Nähe der Enden (39) der Arme (24, 25) angeordnet ist.

16. Maske nach einem der vorhergehenden Ansprüche, **dadurch gekennzeichnet, dass** am Ende (39) mindestens eines der Arme (24, 25) mindestens ein Gasableiter (36) angeordnet ist, um den durch die zusätzlichen Lüftungsdurchlässe (20) geleiteten Gasstrom abzuleiten, wobei die Ableiter (36) vorzugsweise derart angeordnet sind, dass der durch die zusätzlichen Lüftungsdurchlasse (20) geleitete Gasstrom abgeleitet wird, besonders, wenn die Lüftungsdurchlässe (37) verstopft sind.

## Revendications

1. Masque nasal comprenant :
- un élément de base (1), un élément de recouvrement (2) comprenant une ouverture (4) et un élément intermédiaire (3) avec deux buses nasales (5),
- ledit élément intermédiaire (3) étant aménagé de manière étanche entre ledit élément de base (1) et ledit élément de recouvrement (2), les buses nasales (5) faisant saillie vers l'extérieur à travers l'ouverture (4),
- au moins ledit élément intermédiaire (3) et ledit élément de base (1) définissant un volume interne (6) pour le gaz, chacune desdites buses nasales (5) ayant un orifice (11) en communication fluidique via un passage interne (10) avec ledit volume interne (6), et
- des moyens de raccordement (7, 8) pour raccorder l'élément de recouvrement (2) à l'élément de base (1) et maintenir ledit élément de recouvrement (2) d'un seul tenant avec ledit élément de base (1),
**caractérisé en ce que** l'élément intermédiaire (3) comprend un bord périphérique (9) comme moyens d'étanchéité coopérant avec l'élément de base (1) et l'élément de recouvrement (2), ledit bord périphérique (9) dudit élément intermédiaire (3) étant pressé/comprimé entre l'élément de recouvrement (2) et l'élément de base (1).

2. Masque selon la revendication 1, **caractérisé en ce que** l'élément de base (1) comprend une ouverture centrale (12) et deux ouvertures latérales (13, 14), ladite ouverture centrale (12) et les deux ouvertures latérales (13, 14) communiquant avec le volume interne (6).

3. Masque selon la revendication 1 ou la revendication 2, **caractérisé en ce que** l'ouverture centrale (12) et les deux ouvertures latérales (13, 14) ont une taille identique.

4. Masque selon l'une quelconque des revendications précédentes, **caractérisé en ce qu'**une partie (26) de l'élément de base (1) comprend une portion évidée (26) configurée et dimensionnée pour loger au moins une partie du bord d'étanchéité périphérique (9) de l'élément intermédiaire (3).

5. Masque selon l'une quelconque des revendications précédentes, **caractérisé en ce que** le bord périphérique (9) de l'élément intermédiaire (3) est pris en sandwich et pressé/comprimé entre l'élément de recouvrement (2) et la portion évidée (26) de l'élément de base (1).

6. Masque selon l'une quelconque des revendications précédentes, **caractérisé en ce que** l'élément de recouvrement (2) comprend des premiers moyens de raccordement (7) et l'élément de base (1) comprend des seconds moyens de raccordement (8), lesdits premiers et seconds moyens de raccordement (7, 8) coopérant conjointement de manière à maintenir l'élément de recouvrement (2) d'un seul tenant avec au moins ledit élément de base (1).

7. Masque selon l'une quelconque des revendications précédentes, **caractérisé en ce que** :
- un premier élément de fermeture (15) est aménagé dans l'une de ladite ouverture centrale (12) et desdites deux ouvertures latérales (13, 14) de l'élément de base (1) de manière à empêcher toute circulation de gaz entre le volume interne (6) et l'extérieur du masque via ladite ouverture,
- un second élément de fermeture (16) est aménagé dans une autre desdites deux ouvertures latérales (13, 14) de l'élément de base (1) de manière à empêcher toute circulation de gaz entre le volume interne (6) et l'extérieur du masque via ladite ouverture,
- un élément d'alimentation en gaz (17) est raccordé à la dernière de ladite ouverture centrale (12) et desdites deux ouvertures latérales (13, 14) de manière à acheminer du gaz transporté par ledit élément d'alimentation en gaz (17) au volume interne (6).

8. Masque selon l'une quelconque des revendications précédentes, **caractérisé en ce que** ledit élément de fermeture (16) et/ou ledit élément d'alimentation en gaz (17) comprend ou comprennent plusieurs passages d'évacuation (20) aménagés en configuration circulaire autour de la périphérie desdits éléments (16, 17).

9. Masque selon l'une quelconque des revendications précédentes, **caractérisé en ce que** chacun dudit premier élément de fermeture (15), dudit second élément de fermeture (16) et dudit élément d'alimentation en gaz (17) comprend une extrémité de forme tubulaire (21) :
- ayant un diamètre externe légèrement inférieur au diamètre interne de ladite ouverture centrale (12) et desdites deux ouvertures latérales (13, 14) de l'élément de base (1) de manière à être à même d'être inséré dans lesdites ouvertures (12, 13, 14) et
- comprenant des premiers moyens de fixation (22) coopérant avec des seconds moyens de fixation (23) aménagés dans lesdites ouvertures (12, 13, 14) de manière à maintenir chacun desdits éléments (15, 16, 17) en place dans lesdites ouvertures (12, 13, 14) lorsque l'extrémité de forme tubulaire (21) dudit élément (15, 16, 17) est inséré dans l'une desdites ouvertures (12, 13, 14).

10. Masque selon la revendication 9, **caractérisé en ce que** les premiers moyens de fixation (22) et lesdits seconds moyens de fixation (23) comprennent une paroi annulaire et une rainure annulaire coopérant ensemble, de préférence la paroi annulaire (22) est aménagée sur la surface externe de l'extrémité de forme tubulaire (21) et la rainure annulaire (23) est aménagée dans les ouvertures (12, 13, 14).

11. Masque selon l'une quelconque des revendications précédentes, **caractérisé en ce qu'**il comprend en outre un ensemble de couvre-chef (24, 25, 38) raccordé à l'élément de base (1) de manière à maintenir le masque dans une position souhaitée sur la tête d'un utilisateur lorsque les buses nasales (5) sont insérées dans les narines de l'utilisateur.

12. Masque selon l'une quelconque des revendications précédentes, **caractérisé en ce que** l'élément de base (1) est raccordé à l'ensemble de couvre-chef (24, 25, 38) de manière à pouvoir tourner.

13. Masque selon l'une quelconque des revendications précédentes, **caractérisé en ce que** l'ensemble de couvre-chef (24, 25, 38) comprend deux bras allongés (24, 25) chacun raccordés, d'une part, à l'élément de base (1) et, d'autre part, à un couvre-chef (38) comprenant une ou plusieurs sangles ou similaires, qui est positionnée sur la tête de l'utilisateur.

14. Masque selon l'une quelconque des revendications précédentes, **caractérisé en ce que**, dans l'élément de base (1), l'élément de recouvrement (2) et l'élément intermédiaire (3) sont raccordés l'un à l'autre de manière détachable.

15. Masque selon l'une quelconque des revendications précédentes, **caractérisé en ce que** chacun des bras (24, 25) de l'ensemble de couvre-chef (24, 25, 38) comprend une portion flexible (35) située au voisinage des extrémités (39) des bras (24, 25).

16. Masque selon l'une quelconque des revendications précédentes, **caractérisé en ce qu'**au moins un déflecteur de gaz (36) est aménagé à l'extrémité (39) d'au moins l'un des bras (24, 25) pour dévier l'écoulement de gaz évacué à travers les passages d'évacuation supplémentaires (20), les déflecteurs (36) étant aménagés de préférence de manière à dévier l'écoulement de gaz évacué à travers lesdits passages d'évacuation supplémentaires (20), en particulier lorsque les passages d'évacuation (37) sont bouchés.
